## Europäisches Patentamt

## European Patent Office

## Office européen des brevets

(19)

(11) **EP 0 901 016 A2**

(12) **EUROPÄISCHE PATENTANMELDUNG**

(43) Veröffentlichungstag:
10.03.1999 Patentblatt 1999/10

(51) Int. Cl.$^6$: **G01N 33/20**

(21) Anmeldenummer: 98115827.2

(22) Anmeldetag: 21.08.1998

(84) Benannte Vertragsstaaten:
**AT BE CH CY DE DK ES FI FR GB GR IE IT LI LU
MC NL PT SE**
Benannte Erstreckungsstaaten:
**AL LT LV MK RO SI**

(30) Priorität: **05.09.1997 DE 19738943**

(71) Anmelder:
**SIEMENS AKTIENGESELLSCHAFT
80333 München (DE)**

(72) Erfinder:
• **Döll, Rüdiger Dr.
90409 Nürnberg (DE)**
• **Gramckow, Otto Dr.
91052 Erlangen (DE)**

(54) **Verfahren und Einrichtung zur Bestimmung von Eigenschaften eines Stahls**

(57) Verfahren und Einrichtung zur Bestimmung zumindest einer der Größen Streckgrenze, Dehngrenze, Zugfestigkeit, Bruchdehnung, Härte, Duktil-Spröd-Übergangstemperatur und Gefügebestandteile eines Stahls mittels zumindest eines neuronalen Netzes, das zumindest eine der Größen Streckgrenze, Dehngrenze, Zugfestigkeit, Bruchdehnung, Härte, Duktil-Spröd-Übergangstemperatur und Gefügebestandteile in Abhängigkeit von der Zusammensetzung des Stahls oder ausgewählter Kenngrößen über die Zusammensetzung des Stahls und/oder in Abhängigkeit von auf den Stahl einwirkenden Umgebungseinflüssen ermittelt, wobei das neuronale Netz mit zumindest eine der Größen Streckgrenze, Dehngrenze, Zugfestigkeit, Bruchdehnung, Härte, Duktil-Spröd-Übergangstemperatur und Gefügebestandteile, mit Daten wie der Zusammensetzung des Stahls oder ausgewählter Kenngrößen über die Zusammensetzung des Stahls für Stähle, deren Streckgrenze, Dehngrenze, Zugfestigkeit, Bruchdehnung, Härte, Duktil-Spröd-Übergangstemperatur oder Gefügebestandteile bekannt ist, trainiert wird.

FIG 3

EP 0 901 016 A2

**Beschreibung**

Die Erfindung betrifft ein Verfahren sowie eine Einrichtung zur Bestimmung zumindest einer der Größen Streckgrenze, Dehngrenze, Zugfestigkeit, Bruchdehnung, Härte, Duktil-Spröd-Übergangstemperatur und Gefügebestandteile eines Stahls.

Es ist bekannt, bestimmte Eigenschaften, wie z.B. die Streckgrenze $R_e$ oder die Zugfestigkeit $R_m$ eines Stahls durch analytische Zusammenhänge zu bestimmen. Derartige Zusammenhänge sind zum Beispiel bei umlegierten C-Mn-Stählen mit geringem Kohlenstoffanteil:

$$R_e = 88 + 37\,Mn + 83\,Si + 2,3\,f_p + 15,1\,/\sqrt{d_\alpha} \qquad (1)$$

bzw.

$$R_m = 296 + 27,6\,Mn + 82,9\,Si + 3,92\,f_p + 7,81\,/\sqrt{d_\alpha} \qquad (2)$$

Dabei ist

Mn   der Mangananteil im Stahl
Si   der Siliziumanteil im Stahl
$f_p$   der Perlitanteil im Stahl
$d_\alpha$   die Ferritkorngröße im Stahl

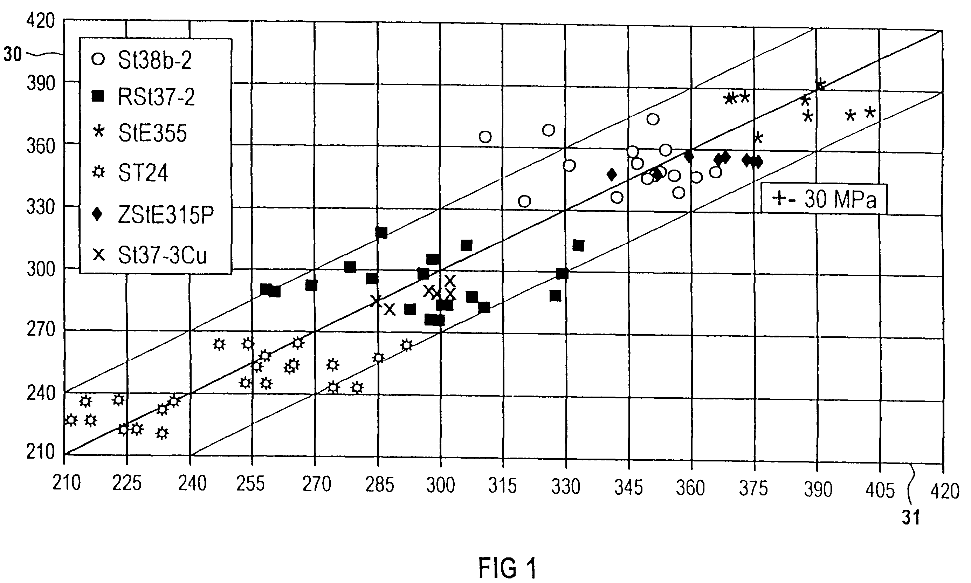

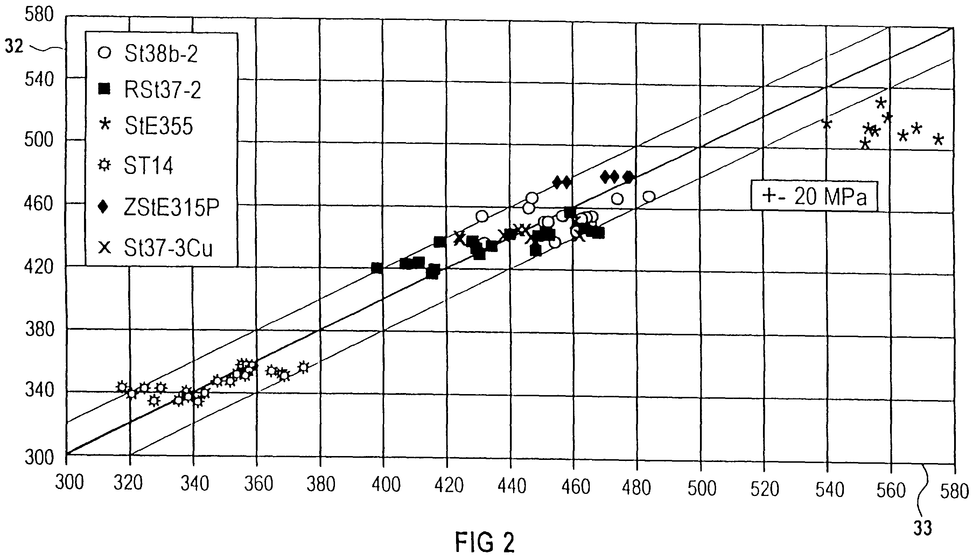

Derartige Zusammenhänge führen jedoch häufig nicht zu der gewünschten Präzision bei der Bestimmung der gewünschten Größen. So zeigen z.B. FIG 1 und FIG 2 die Differenz zwischen berechneten und gemessenen Werten für die Streckgrenze bzw. die Zugfestigkeit für die Stähle St38b-2, RSt37-2, StE355, St14, ZStE315P und St37-3Cu. Dabei bezeichnet Achse 30 den berechneten Wert der Streckgrenze in MPa, Achse 31 den gemessenen Wert der Streckgrenze in MPa, Achse 32 den berechneten Wert für die Zugfestigkeit in MPa und Achse 33 den gemessenen Wert für die Zugfestigkeit in MPa. FIG 1 und 2 zeigen dabei deutlich, daß für die Präzision der berechneten Werte allenfalls eine Garantie von ± 30 MPa bzw. ± 20 MPa gegeben werden kann, was bei hohen Qualitätsanforderungen nicht ausreichend ist. Außerdem sind für manche Größen (z.B. Bruchdehnung) und manche Stahlgüten (z.B. legierte Stähle) keine analytischen Zusammenhänge bekannt, und es muß zur Bestimmung der entsprechenden Größen auf Meßwerte oder sehr ungenaue Erfahrungswerte zurückgegriffen werden.

Entsprechend ist es Aufgabe der Erfindung, ein Verfahren bzw. eine Einrichtung zur Durchführung des Verfahrens anzugeben, das eine Abschätzung zumindest einer der Größen Streckgrenze, Dehngrenze, Zugfestigkeit, Bruchdehnung, Härte, Duktil-Spröd-Übergangstemperatur und Gefügebestandteile von Stählen mit größerer Präzision als mit den bekannten Verfahren ermöglicht.

Die Aufgabe wird erfindungsgemäß durch ein Verfahren gemäß Anspruch 1 bzw. Einrichtung gemäß Anspruch 14 gelöst. Ein derartiges neuronales Netz hat sich überraschend gut bewährt, um die stark nicht linearen Einflüsse von Größen wie Temperatur, Formänderungsgeschwindigkeit, Umformgrad oder die Zusammensetzung des Stahls auf zumindest eine der Größen Streckgrenze, Dehngrenze, Zugfestigkeit, Bruchdehnung, Härte, Duktil-Spröd-Übergangstemperatur und Gefügebestandteile zu ermitteln.

In einer vorteilhaften Ausgestaltung der Erfindung bestimmt das neuronale Netz zumindest eine der Größen Streckgrenze, Dehngrenze, Zugfestigkeit, Bruchdehnung, Härte, Duktil-Spröd-Übergangstemperatur und Gefügebestandteile des Stahls in Abhängigkeit der Temperatur, des Umformgrades bzw. der relativen Umformung des Stahls, der Umformgeschwindigkeit sowie der Legierungsanteile und ggf. der Korngröße im Gefüge des Stahls.

Für eine einfache Konfiguration des neuronalen Netzes hat es sich als besonders vorteilig erwiesen, dem neuronalen Netz zur Bestimmung zumindest eine der Größen Streckgrenze, Dehngrenze, Zugfestigkeit, Bruchdehnung, Härte, Duktil-Spröd-Übergangstemperatur und Gefügebestandteile den Kohlenstoffanteil und den Mangananteil im Stahl als Eingangsgrößen zuzuführen.

In einer weiteren vorteilhaften Ausgestaltung des erfindungsgemäßen Verfahrens bestimmt das neuronale Netz zumindest eine der Größen Streckgrenze, Dehngrenze, Zugfestigkeit, Bruchdehnung, Härte, Duktil-Spröd-Übergangstemperatur und Gefügebestandteile des zu untersuchenden Stahls in Abhängigkeit der einzelnen Legierungsanteile im Stahl. Dabei hat es sich als besonders vorteilhaft herausgestellt, zumindest eine der Größen Streckgrenze, Dehngrenze, Zugfestigkeit, Bruchdehnung, Härte, Duktil-Spröd-Übergangstemperatur und Gefügebestandteile in Abhängigkeit vom Kohlenstoffanteil, vom Siliziumanteil, vom Mangananteil, vom Phosphoranteil, vom Schwefelanteil, vom Kobaltanteil, vom Aluminiumanteil, vom Chromanteil, vom Molybdänanteil, vom Nickelanteil, vom Vanadiumanteil, vom Kupferanteil, vom Zinnanteil, vom Calziumanteil, vom Titananteil, vom Boranteil, vom Niobanteil, vom Arsenanteil, vom Wolframanteil und vom Stickstoffanteil zu bestimmen.

Es ist besonders vorteilhaft, das neuronale Netz im Rahmen der Prozeßautomatisierung einzusetzen. Stehen z. B. der Prozeßautomatisierung einer Walzstraße die zur Durchführung des erfindungsgemäßen Verfahrens notwendigen Eingangsinformationen zur Verfügung, so kann diese selbsttätig das erfindungsgemäße Verfahren durchführen, so daß in der Prozeßautomatisierung zumindest eine der Größen Streckgrenze, Dehngrenze, Zugfestigkeit, Bruchdehnung, Härte, Duktil-Spröd-Übergangstemperatur und Gefügebestandteile des zu bearbeitenden Stahls zur Verfügung steht.

Für ein neuronales Netz, dem u.a. verschiedene Legierungsanteile als Eingangsgrößen zugeführt werden, so daß dieses auf 15 oder mehr Eingangsgrößen kommt, hat es sich als vorteilhaft erwiesen, es als Multilayer Perceptron mit einer verdeckten Ebene, die 80 bis 120, vorteilhafterweise 100 Knoten aufweist, auszuführen.

In einer weiteren vorteilhaften Ausgestaltung des erfindungsgemäßen Verfahrens wird das neuronale Netz mit der Backpropagation Methode trainiert.

In einer weiteren vorteilhaften Ausgestaltung des erfindungsgemäßen Verfahrens ist es als Expertensystem implementiert.

In einer weiteren vorteilhaften Ausgestaltung des erfindungsgemäßen Verfahrens ist das neuronale Netz als Multilayer Perceptron mit einer verdeckten Ebene ausgebildet.

Weitere Vorteile und Einzelheiten ergeben sich aus der nachfolgenden Beschreibung von Ausführungsbeispielen, anhand der Zeichnungen und in Verbindung mit den Unteransprüchen. Im einzelnen zeigen:

FIG 1 Zusammenhang zwischen gemessenen und berechneten Werten für die Streckgrenze verschiedener Stähle
FIG 2 Zusammenhang zwischen gemessenen und berechneten Werten für die Zugfestigkeit verschiedener Stähle
FIG 3 ein einfaches erfindungsgemäßes neuronales Netz
FIG 4 ein einfaches erfindungsgemäßes neuronales Netz in erweiterter Konfiguration
FIG 5 ein komplexes erfindungsgemäßes neuronales Netz
FIG 6 ein komplexes erfindungsgemäßes neuronales Netz in erweiterter Konfiguration.

FIG 1 und FIG 2 zeigen die Differerenz zwischen berechneten und gemessenen Werten für Streckgrenze $R_e$ bzw. Zugfestigkeit $R_m$ für die Stähle St38b-2, RSt37-2, StE355, St14, ZStE315P und St37-3Cu, wobei die Werte für die Streckgrenze $R_e$ bzw. die Zugfestigkeit $R_m$ gemäß

$$R_e = 88 + 37\ \text{Mn} + 83\ \text{Si} + 2,3\ f_p + 15,1\ /\sqrt{d_\alpha} \tag{3}$$

bzw.

$$R_m = 296 + 27,6\ \text{Mn} + 82,9\ \text{Si} + 3,92\ f_p + 7,81\ /\sqrt{d_\alpha} \tag{4}$$

berechnet sind, wobei Mn der Mangananteil im Stahl, Si der Siliziumanteil im Stahl, $f_p$ der Perlitanteil im Stahl und $d_\alpha$ die Ferritkorngröße ist. Dabei bezeichnet Achse 30 den berechneten Wert der Streckgrenze in MPa, Achse 31 den gemessenen Wert der Streckgrenze in MPa, Achse 32 den berechneten Wert für die Zugfestigkeit in MPa und Achse 83 den gemessenen Wert für die Zugfestigkeit in MPa. FIG 1 und 2 zeigen, daß für die Präzision der, gemäß dem bekannten Verfahren, berechneten Werte allenfalls eine Garantie von ± 30 MPa bzw. ± 20 MPa gegeben werden kann. Dies ist bei hohen Qualitätsanforderungen nicht ausreichend.

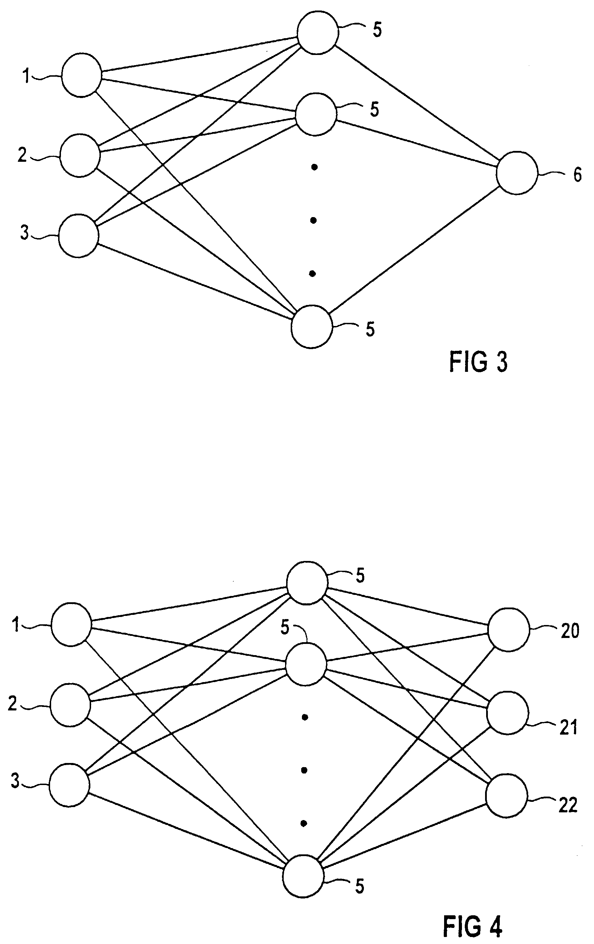

FIG 3 zeigt ein einfaches erfindungsgemäßes neuronales Netz zur Bestimmung zumindest einer der Größen Streckgrenze, Dehngrenze, Zugfestigkeit, Bruchdehnung, Härte, Duktil-Spröd-Übergangstemperatur und Gefügebestandteile eines Stahls. Das Netz ermittelt zumindest eine der Größen Streckgrenze, Dehngrenze, Zugfestigkeit, Bruchdehnung, Härte, Duktil-Spröd-Übergangstemperatur und Gefügebestandteile des Stahls in Abhängigkeit vom Umformgrad 1 des Stahls an den einzelnen Gerüsten einer Walzenstraße von der Endwalz- und Haspeltemperatur 2 und 3 des Stahls und vom Kohlenstoffanteil 4 im Stahl. Das neuronale Netz weist eine Ebene mit verdeckten Neuronen 5 auf. Ist vorgesehen, mehrere der Größen Streckgrenze, Dehngrenze, Zugfestigkeit, Bruchdehnung, Härte, Duktil-Spröd-Übergangstemperatur und Gefügebestandteile zu bestimmen, so wird gemäß vorteilhafter Ausgestaltung der Erfindung für jede dieser Größen ein neuronales Netz vorgesehen. Sollen mehrere der Größen Streckgrenze, Dehngrenze, Zugfestigkeit, Bruchdehnung, Härte, Duktil-Spröd-Übergangstemperatur und Gefügebestandteile des Stahls ermittelt werden, so werden also entsprechend viele neuronale Netze verwendet, wobei jedes neuronale Netz eine der Größen Streckgrenze, Dehngrenze, Zugfestigkeit, Bruchdehnung, Härte, Duktil-Spröd-Übergangstemperatur und Gefügebestandteile ermittelt.

Eine alternative Ausgestaltung zu diesem vorteilhaften Vorgehen zeigt FIG 4, wobei Bezugszeichen 1 bis 5 die gleiche Bedeutung haben wie in FIG 3. Das neuronale Netz gemäß FIG 4 weist beispielhaft drei Ausgangsknoten 20, 21, 22 auf. In beispielhafter Ausgestaltung liefern die Ausgangsknoten 20, 21, bzw. 22 Werte für die Größen Streckgrenze, Zugfestigkeit und Bruchdehnung. Sollen mehr als drei der Größen Streckgrenze, Zugfestigkeit und Bruchdeh-

EP 0 901 016 A2

nung ermittelt werden, so ist die Anzahl der Ausgangsknoten entsprechend zu erhöhen. Sollen z.B. zusätzlich Härte und Duktil-Spröd-Übergangstemperatur bestimmt werden, so ist ein neuronales Netz mit fünf Ausgangsknoten vorzusehen. In alternativer Ausgestaltung dazu ist z.B. ein Netz mit drei Ausgangsknoten zur Ermittlung der Größen Streckgrenze, Zugfestigkeit und Bruchdehnung und ein zweites neuronales Netz mit zwei Ausgangsknoten zur Ermittlung der Härte und Duktil-Spröd-Übergangstemperatur vorzusehen.

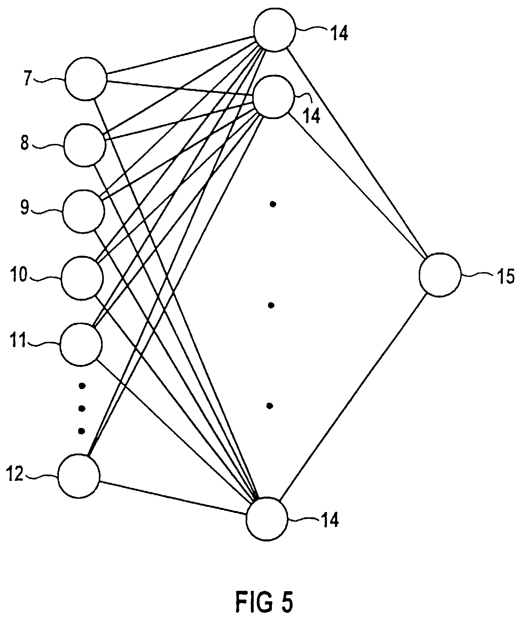

FIG 5 zeigt ein komplexes erfindungsgemäßes neuronales Netz zur Bestimmung der Größen Streckgrenze, Dehngrenze, Zugfestigkeit, Bruchdehnung, Härte, Duktil-Spröd-Übergangstemperatur und Gefügebestandteile eines Stahls. Dabei wird eine der Größen 15 Streckgrenze, Dehngrenze, Zugfestigkeit, Bruchdehnung, Härte, Duktil-Spröd-Übergangstemperatur und Gefügebestandteile in Abhängigkeit vom Umformgrad 7 des Stahls an den einzelnen Gerüsten einer Walzstraße, der Umformgeschwindigkeit 8 in den einzelnen Gerüsten der Walzstraße, der Endwalz- und Haspeltemperatur 9 des Stahls, vom Kohlenstoffanteil 10, vom Siliziumanteil 11, vom Mangananteil, vom Phosphoranteil, vom Schwefelanteil, vom Kobaltanteil, vom Aluminiumanteil, vom Chromanteil, vom Molyodänanteil, vom Nickelanteil, vom Vanadiumanteil, vom Kupferanteil, vom Zinnanteil, vom Calziumanteil, vom Titananteil, vom Boranteil, vom Niobanteil, vom Arsenanteil, vom Wolframanteil und vom Stickstoffanteil 12 ermittelt. Das neuronale Netz weist vorteilhafterweise eine Ebene mit 100 verdeckten Neuronen 14 auf. Sollen mehrere der Größen Streckgrenze, Dehngrenze, Zugfestigkeit, Bruchdehnung, Härte, Duktil-Spröd-Übergangstemperatur und Gefügebestandteile des Stahls ermittelt werden, so werden entsprechend viele neuronale Netze verwendet, wobei jedes neuronale Netz eine der Größen Streckgrenze, Dehngrenze, Zugfestigkeit, Bruchdehnung, Härte, Duktil-Spröd-Übergangstemperatur und Gefügebestandteile ermittelt.

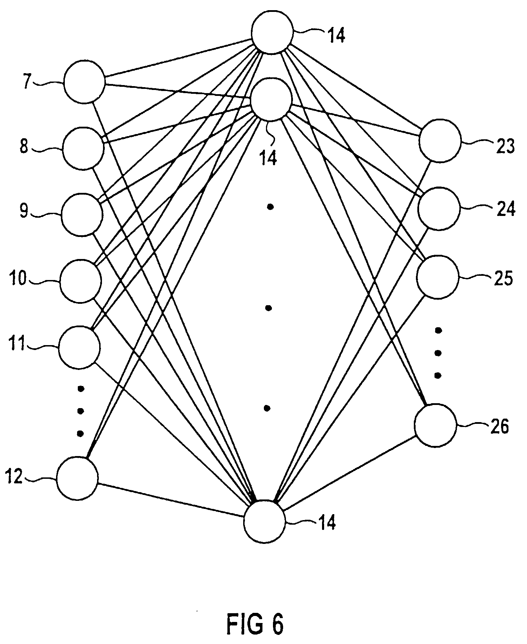

FIG 6 zeigt ein komplexes erfindungsgemäßes neuronales Netz in alternativer Ausgestaltung. Dabei haben die Bezugszeichen 7 bis 14 die gleiche Bedeutung wie in FIG 5. Im Gegensatz zu dem neuronalen Netz in FIG 5, ermittelt das neuronale Netz in FIG 6 mehrere der Größen Streckgrenze, Dehngrenze, Zugfestigkeit, Bruchdehnung, Härte, Duktil-Spröd-Übergangstemperatur und Gefügebestandteile des Stahls. Die entsprechenden Werte liegen an den Ausgangsknoten 23,24,25,26 an.

Für eine präzise Bestimmung der Größen Streckgrenze, Dehngrenze, Zugfestigkeit, Bruchdehnung, Härte, Duktil-Spröd-Übergangstemperatur und Gefügebestandteile hat es sich als besonders vorteilhaft erwiesen, neben bestimmten Legierungsanteilen, insbesondere Kohlenstoff, Stickstoff, Silizium und Mangan, walzspezifische Parameter als Eingangsgrößen in das neuronale Netz zu verwenden. Als besonders wichtige und vorteilhafte walzspezifische Parameter haben sich der Temperaturverlauf und die Umformgrade an den Walzgerüsten erwiesen. Zur weiteren Erhöhung der Präzision bei der Bestimmung der Größen Streckgrenze, Dehngrenze, Zugfestigkeit, Bruchdehnung, Härte, Duktil-Spröd-Übergangstemperatur und Gefügebestandteile werden zusätzlich Bandgeschwindigkeiten, Walzstraßengeometrie, Walzkräfte und oder Walzmomente als Eingangsgrößen verwendet. Es hat sich weiterhin als vorteilhaft erwiesen, den Temperaturverlauf des Stahls über seine gesamte Herstellung, d.h., auch vor dem Walzprozeß, wie z.B. beim Stranggießen, als Eingangsgröße des neuronalen Netzes zu verwenden.

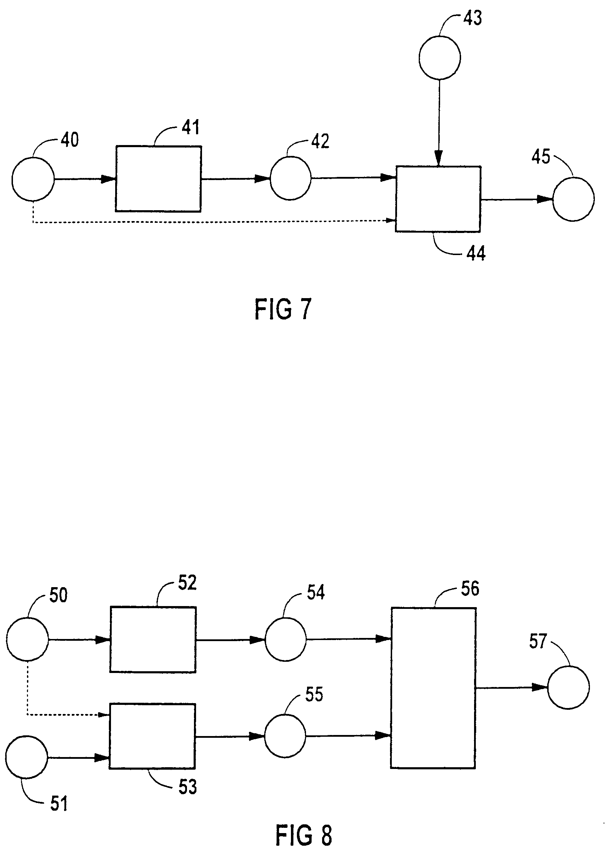

Neben der direkten Bestimmung der Größen Streckgrenze, Dehngrenze, Zugfestigkeit, Bruchdehnung, Härte, Duktil-Spröd-Übergangstemperatur und Gefügebestandteile mittels der neuronalen Netze, wie sie z.B. FIG 3 bis 6 beispielhaft zeigen, ist es ebenfalls vorteilhaft, an Stelle der Größen Streckgrenze, Dehngrenze, Zugfestigkeit, Bruchdehnung, Härte, Duktil-Spröd-Übergangstemperatur und Gefügebestandteile Korrekturfaktoren für diese Größen zu ermitteln. Dabei werden analytische Zusammenhänge, wie sie z.B. Gleichungen 3 und 4 zeigen, mittels eines neuronalen Netzes korrigiert. Ein entsprechendes Vorgehen zeigt dabei FIG 7. Dabei werden aus bestimmten Eingangsgrößen 40 mittels eines analytischen Zusammenhangs 41 vorläufige Werte 42 für Streckgrenze, Dehngrenze, Zugfestigkeit, Bruchdehnung, Härte Duktil-Spröd-Übergangstemperatur und/oder Gefügebestandteile gebildet. Diese Werte 42 werden zusammen mit weiteren Eingangsgrößen 43 sowie optional den Eingangsgrößen 40 bzw. einer Teilmenge der Eingangsgrößen 40 am neuronalen Netz 44 zugeführt, daß aus diesen Größen 40,43 Werte 45 für Streckgrenze, Dehngrenze, Zugfestigkeit, Bruchdehnung, Härte, Duktil-Spröd-Übergangstemperatur und/oder Gefügebestandteile errechnet.

Eine alternative Vorgehensweise der Berechnung der Größen Streckgrenze, Dehngrenze, Zugfestigkeit, Bruchdehnung, Härte, Duktil-Spröd-Übergangstemperatur und Gefügebestandteile gemäß FIG 7 zeigt FIG 8. Dabei wird mittels eines analytischen Zusammenhangs 52 aus bestimmten Eingangsgrößen 50 vorläufige Werte 54 für Streckgrenze, Dehngrenze, Zugfestigkeit, Bruchdehnung, Härte, Duktil-Spröd-Übergangstemperatur und/oder Gefügebestandteile ermittelt. In einem Parallelzweig werden mittels eines neuronalen Netzes 53 aus weiteren Eingangsgrößen 51 sowie optional aus den Eingangsgrößen 50 oder einer Teilmenge der Eingangsgrößen 50 Korrekturfaktoren 55 für Streckgrenze, Dehngrenze, Zugfestigkeit, Bruchdehnung, Härte, Duktil-Spröd-Übergangstemperatur und/oder Gefügebestandteile. Die vorläufigen Werte 54 für Streckgrenze, Dehngrenze, Zugfestigkeit, Bruchdehnung, Härte, Duktil-Spröd-Übergangstemperatur und/oder Gefügebestandteile und die entsprechenden Korrekturwerte 55 werden einem Verknüpfungsblock 56 zu Werten 57 für Streckgrenze, Dehngrenze, Zugfestigkeit, Bruchdehnung, Härte, Duktil-Spröd-Übergangstemperatur und/oder Gefügebestandteile verknüpft. Der Funktionsblock 56 verknüpft die Werte 54 und 55 additiv oder vorteilhafterweise multiplikativ. Die Eingangsgrößen 40,43,50 und 51 in FIG 7 und 8 entsprechen den Eingangsgrößen 1,2,3,7,8,9,10,11,12 in FIG 3 bis 6 bzw. einer Auswahl dieser Größen.

## Patentansprüche

1. Verfahren zur Bestimmung zumindest einer der Größen Streckgrenze, Dehngrenze, Zugfestigkeit, Bruchdehnung, Härte, Duktil-Spröd-Übergangstemperatur und Gefügebestandteile eines Stahls mittels zumindest eines neuronalen Netzes, das zumindest eine der Größen Streckgrenze, Dehngrenze, Zugfestigkeit, Bruchdehnung, Härte, Duktil-Spröd-Übergangstemperatur und Gefügebestandteile in Abhängigkeit von der Zusammensetzung des Stahls oder ausgewählter Kenngrößen über die Zusammensetzung des Stahls und/oder in Abhängigkeit von auf den Stahl einwirkenden Umgebungseinflüssen ermittelt, wobei das neuronale Netz mit zumindest einer der Größen Streckgrenze, Dehngrenze, Zugfestigkeit, Bruchdehnung, Härte, Duktil-Spröd-Übergangstemperatur und Gefügebestandteile, mit Daten wie der Zusammensetzung des Stahls oder ausgewählter Kenngrößen über die Zusammensetzung des Stahls für Stähle, deren Streckgrenze, Dehngrenze, Zugfestigkeit, Bruchdehnung, Härte, Duktil-Spröd-Übergangstemperatur oder Gefügebestandteile bekannt ist, trainiert wird.

2. Verfahren nach Anspruch 1,
   **dadurch gekennzeichnet**, daß das neuronale Netz mit Daten wie auf den Stahl einwirkende Umgebungseinflüsse für Stähle, deren Streckgrenze, Dehngrenze, Zugfestigkeit, Bruchdehnung, Härte, Duktil-Spröd-Übergangstemperatur und Gefügebestandteile bekannt ist, trainiert wird.

3. Verfahren nach Anspruch 1 oder 2,
   **dadurch gekennzeichnet**, daß zumindest eine der Größen Streckgrenze, Dehngrenze, Zugfestigkeit, Bruchdehnung, Härte, Duktil-Spröd-Übergangstemperatur und Gefügebestandteile in Abhängigkeit der Endwalztemperatur und/oder der Haspeltemperatur des Stahls ermittelt wird.

4. Verfahren nach Anspruch 1, 2 oder 3,
   **dadurch gekennzeichnet**,
   daß zumindest eine der Größen Streckgrenze, Dehngrenze, Zugfestigkeit, Bruchdehnung, Härte, Duktil-Spröd-Übergangstemperatur und Gefügebestandteile in Abhängigkeit der Umformbedingungen, z. B. in Abhängigkeit vom Umformgrad oder der relativen Umformung, ermittelt wird.

5. Verfahren nach Anspruch 4,
   **dadurch gekennzeichnet**,
   daß zumindest eine der Größen Streckgrenze, Dehngrenze, Zugfestigkeit, Bruchdehnung, Härte, Duktil-Spröd-Übergangstemperatur und Gefügebestandteile in Abhängigkeit von der Umformgeschwindigkeit ermittelt wird.

6. Verfahren nach einem der Ansprüche 1 bis 5,
   **dadurch gekennzeichnet**,
   daß zumindest eine der Größen Streckgrenze, Dehngrenze, Zugfestigkeit, Bruchdehnung, Härte, Duktil-Spröd-Übergangstemperatur und Gefügebestandteile in Abhängigkeit vom Kohlenstoffanteil, vom Mangananteil und vom Siliziumanteil im Stahl ermittelt wird.

7. Verfahren nach einem der Ansprüche 1 bis 6,
   **dadurch gekennzeichnet**,
   daß zumindest eine der Größen Streckgrenze, Dehngrenze, Zugfestigkeit, Bruchdehnung, Härte, Duktil-Spröd-Übergangstemperatur und Gefügebestandteile in Abhängigkeit vom Siliziumanteil, vom Mangananteil, vom Phosphoranteil, vom Schwefelanteil, vom Kobaltanteil, vom Aluminiumanteil, vom Chromanteil, vom Molyodänanteil, vom Nickelanteil, vom Vanadiumanteil, vom Kupferanteil, vom Zinnanteil, vom Calziumanteil, vom Titananteil, vom Boranteil, vom Niobanteil, vom Arsenanteil, vom Wolframanteil und vom Stickstoffanteil oder einer Kombination dieser Anteile sowie Endwalz- und Haspeltemperatur ermittelt wird.

8. Verfahren nach einem der Ansprüche 1 bis 7,
   **dadurch gekennzeichnet**,
   daß zumindest eine der Größen Streckgrenze, Dehngrenze, Zugfestigkeit, Bruchdehnung, Härte, Duktil-Spröd-Übergangstemperatur und Gefügebestandteile in Abhängigkeit vom Eisenanteil im Stahl ermittelt wird.

9. Verfahren nach einem der Ansprüche 1 bis 8,
   **dadurch gekennzeichnet**,

daß zumindest eine der Größen Streckgrenze, Dehngrenze, Zugfestigkeit, Bruchdehnung, Härte, Duktil-Spröd-Übergangstemperatur und Gefügebestandteile in Abhängigkeit von der Gefügestruktur des Stahls ermittelt wird.

10. Verfahren nach einem der Ansprüche 1 bis 9,
**dadurch gekennzeichnet**,
daß zumindest eine der Größen Streckgrenze, Dehngrenze, Zugfestigkeit, Bruchdehnung, Härte, Duktil-Spröd-Übergangstemperatur und Gefügebestandteile in Abhängigkeit von walzspezifischen Parametern beim Walzen des Stahls, insbesondere in Abhängigkeit zumindest einer der Größen Temperaturverlauf, Umformgrade an den Walzgerüsten Bandgeschwindigkeiten, Walzstraßengeometrie, Walzkräfte und Walzmomente, ermittelt wird.

11. Verfahren nach einem der Ansprüche 1 bis 10,
**dadurch gekennzeichnet**,
daß zumindest eine der Größen Streckgrenze, Dehngrenze, Zugfestigkeit, Bruchdehnung, Härte, Duktil-Spröd-Übergangstemperatur und Gefügebestandteile in Abhängigkeit von Temperaturverlauf des Stahls bei der Herstellung des Stahls ermittelt wird.

12. Verfahren nach einem der Ansprüche 1 bis 11,
**dadurch gekennzeichnet**,
daß zumindest eine der Größen Streckgrenze, Dehngrenze, Zugfestigkeit, Bruchdehnung, Härte, Duktil-Spröd-Übergangstemperatur und Gefügebestandteile in Abhängigkeit von der Gefügestruktur des Stahls ermittelt wird.

13. Verfahren nach einem der Ansprüche 1 bis 12,
**dadurch gekennzeichnet**,
daß als Trainingsdaten für das neuronale Netz tabellarisch abgelegte Daten über zumindest eine der Größen Streckgrenze, Dehngrenze, Zugfestigkeit, Bruchdehnung, Härte, Duktil-Spröd-Übergangstemperatur und Gefügebestandteile bestimmter Stähle, insbesondere aus der Literatur bekannte Daten, verwendet werden.

14. Einrichtung zur Durchführung des Verfahrens nach einem der vorhergehenden der Ansprüche zur Bestimmung zumindest einer der Größen Streckgrenze, Dehngrenze, Zugfestigkeit, Bruchdehnung, Härte, Duktil-Spröd-Übergangstemperatur und Gefügebestandteile eines Stahls mittels zumindest eines auf einer Datenverarbeitungseinrichtung ablaufenden neuronalen Netzes, das zumindest eine der Größen Streckgrenze, Dehngrenze, Zugfestigkeit, Bruchdehnung, Härte, Duktil-Spröd-Übergangstemperatur und Gefügebestandteile in Abhängigkeit von der Zusammensetzung des Stahls oder ausgewählter Kenngrößen über die Zusammensetzung des Stahls und/oder in Abhängigkeit von auf den Stahl einwirkenden Umgebungseinflüssen ermittelt, wobei das neuronale Netz mit zumindest einer der Größen Streckgrenze, Dehngrenze, Zugfestigkeit, Bruchdehnung, Härte, Duktil-Spröd-Übergangstemperatur und Gefügebestandteile, mit Daten wie der Zusammensetzung des Stahls oder ausgewählter Kenngrößen über die Zusammensetzung des Stahls und/oder mit Daten wie auf den Stahl einwirkende Umgebungseinflüsse für Stähle, deren Streckgrenze, Dehngrenze, Zugfestigkeit, Bruchdehnung, Härte, Duktil-Spröd-Übergangstemperatur oder Gefügebestandteile bekannt ist, trainiert wird.

FIG 1

FIG 2

EP 0 901 016 A2

FIG 3

FIG 4

FIG 5

FIG 6

FIG 7

FIG 8